# EUROPEAN PATENT SPECIFICATION

(11) **EP 2 857 379 B1**
(45) Date of publication and mention of the grant of the patent: **02.09.2020**
(21) Application number: 13787858.3
(22) Date of filing: 08.05.2013
(51) Int. Cl.: C07C 49/255, C07C 45/74, A61K 31/121, G03F 7/004

(54) **METHOD FOR THE SYNTHESIS OF CURCUMIN**
VERFAHREN ZUR SYNTHESE VON CURCUMIN
PROCÉDÉ DE SYNTHÈSE DE CURCUMINE

(30) Priority: 08.05.2012 EP 12385001
(43) Date of publication of application: 08.04.2015
(73) Proprietor: Asac Compañía De Biotecnología E Investigación SA, 03006 Alicante (ES)
(72) Inventor: SOTO SALVADOR, Javier, 03690 San Vicente del Raspeig (Alicante) (ES); YUS ASTIZ, Miguel, 03690 San Vicente del Raspeig (Alicante) (ES)
(74) Representative: Pardo Zapata, José
(86) International application number: PCT/ES2013/000113
(87) International publication number: WO 2013/167770

(56) References cited:
- EP-B1- 0 861 223
- WO-A1-2006/089894
- WO-A1-2007/110168
- DE-A1- 2 501 220
- NURFINA, A.N. ET AL.: 'Synthesis of some symmetrical curcumin derivatives and their antiinflammatory activity' EUROPEAN JOURNAL OF MEDICINAL CHEMISTRY vol. 32, 1997, pages 321 - 328, XP005276597
- PARK, K. & LEE, J.-H: 'Photosensitizer effect of curcumin on UVB-irradiated HaCaT cells through activation of caspase pathways' ONCOLOGY REPORTS vol. 17, 2007, pages 537 - 540, XP055176322

## Description

### Field of the invention

The invention describes a process for obtaining curcumin, characterised in that the boron-curcumin complex formed after the condensation is isolated before its hydrolysis. The invention describes curcumin monohydrate and processes for conversion of curcumin monohydrate to anhydrous curcumin and vice versa.

### Background of the invention

Curcumin is a yellow pigment extracted from the rhizomes of Curcuma Longa. This compound has shown a large number of pharmacological properties, especially when administered concomitantly with visible - ultraviolet radiation as described in EP2236150.

Obtaining curcumin from *Curcuma longa* rhizomes has various disadvantages: presence of pesticides and radioactivity, difference in yield and the presence of other curcuminoids. Therefore, large scale chemical synthesis is advisable.

Industrial scale synthesis must use inexpensive reagents, use few steps, ensure that the products can be handled on a large scale and that the process is robust.

Curcumin synthesis routes described in the state of the art comprise hydrolysis of a boron-curcumin complex obtained by reaction of vanillin in an organic solvent with acetylacetone, boron oxide and an alkyl borate in the presence of a catalytic amount of an alkylamine, as described in US5679864, WO9716403 or US7507864 and in Liebigs Ann Chem 1557-1569, 1985 ; JCS Perkin Trans I, I, 2379-2388, 1972.

The document most similar to the invention, DE 2501220, describes a method for obtaining curcumin by hydrolysis of boric esters of curcumin, where the solvent used in the formation of boric esters and water were removed by azeotropic evaporation. However, WO2007/110168 advises against including an additional step in the synthesis to isolate boron-curcumin complexes before hydrolysis because the yields are not attractive.

There are various solvents used in the state of the art for final purification of curcumin, but there are no technical differences between them. Curcumin obtained after separation and purification is presented in crystalline form with a melting point around 179-183 °C. Typical yields described in the state of the art for the synthesis of curcumin are around 60-65%.

EP 861223 describes in example 1, the synthesis of 136 grams of curcumin and the raw product appears as a heavy oil that is later converted into a crystalline solid. Similarly, WO2007/110168 describes that the product of the reaction after treatment with water/acetic acid is presented as a slurry. The slurries described in the state of the art cannot be handled when the process is performed on an industrial scale and the conditions of the reaction must be modified.
Synthesis processes at industrial scale must be robust, that is, small modifications in the amount of reagents and/or working conditions should not alter the product specifications: residual solvents, sulphuric ash, heavy metals, etc.

The salt and ion concentration in drinking water can be expressed by various parameters such as water hardness, conductivity, resistance, calcium carbonate content and dry residue. The most frequently used parameter is French degrees (°F) equivalent to 10 mg/l of calcium carbonate in water. The hardness of drinking water depends on the source of supply.

Finally, an expert in the subject knows that modifying the crystallisation conditions of a molecule can give different polymorphs or solvates that have different solubilities.

Obtaining new polymorphs or solvates for developing pharmaceutical formulations with new pharmacotechnical properties is of special interest.

### Object of the invention

The problem resolved by the invention is obtaining a reproducible and robust method for synthesising curcumin on an industrial scale, with batch sizes greater than 1 kg and with a sulphuric ash content less than 20 ppm.

The solution found by the inventors, according to claim 1, is separating the precipitate formed by the condensation of vanillin with acetylacetone by filtration before hydrolysing it in aqueous acidic medium.

Surprisingly, including an additional step in the synthesis does not reduce yields, which are maintained around 60-65%, but also prevents the formation of slurries during the crystallisation of curcumin, facilitating product handling when performed on an industrial scale.

Another problem resolved by the invention is reducing the cost of synthesis. If drinking water is used, the costs of purifying the water by distillation, osmosis or ion exchange are avoided and the manipulation time of the slurries formed is also reduced.

In other aspects, the invention describes curcumin hydrate and processes for the conversion of curcumin monohydrate to anhydrous curcumin and vice versa.

### Detailed description of the invention

The synthesis route used in this invention comprises preparation of a suspension of alkyl borate, butylamine and vanillin in ethyl acetate; heating the suspension to 70 °C; adding drop by drop to the suspension and allowing reaction to proceed at 70 °C overnight; filtering the solid precipitated when the heating is turned off; hydrolysing the precipitated solid with an acidic solution; filtering the washed solid and recrystallising in organic solvents.

In a preferred embodiment, the alkyl borate is tributyl borate; the hydrolysis is performed with acetic acid/water and crystallisation of curcumin is performed in ethyl acetate or in acetone/water.

The synthesis route differs from those described in the state of the art by the inclusion of an additional operation: filtration of the precipitated boron complexes solids formed in order to remove the solvents and secondary products.

Synthesis of curcumin on laboratory scale (250 grams) without filtering out the boron complexes produces, after hydrolysis, a viscous slurry similar to that described in EP 861223 or WO2007110168, which after crystallisation with acetone/water leads to anhydrous curcumin. The sulphuric ash content of the product obtained is greater than 20 ppm. Consequently, synthesis of curcumin without performing the filtration of the boron complex precipitate is not viable at the industrial scale because the product cannot be handled, and also the curcumin obtained does not meet the specifications.

Recrystallisation of the curcumin obtained above with a sulphuric ash content over 20 ppm in acetone/drinking water produces curcumin monohydrate, not described above.

To obtain 8 kg curcumin, when hydrolysis of the boron complex is performed after its filtration and not in the reaction mixture, one obtains not an oil but a crystalline solid with a sulphuric ash content less than 20 ppm, which when recrystallised in acetone/drinking water yields anhydrous curcumin.

Recrystallisation of anhydrous curcumin in acetone/water depends on the type of water and sulphuric ash content, as detailed below.

| Batch | Heavy metals | Water type | Final product |
|---|---|---|---|
| 9/2011 | >20 ppm | Drinking* | Hydrate |
| 9/2011 | >20 ppm | by osmosis | Anhydrous |
| 9/2011 | >20 ppm | Ultrapure | Hydrate |
| 7/2011 | <20 ppm | Drinking* | Anhydrous |
| 2/2012 | <20 ppm | Drinking* | Anhydrous |
| 2/2012 | <20 ppm | Borated** | Hydrate |
| 2/2012 | <20 ppm | Saline*** | Hydrate |

| | | | |
|---|---|---|---|
| * Water hardness 20-40 °F ** Drinking water doped with 62 ppm of boron (III). *** Drinking water with 5% sodium chloride. | | | |

Recrystallisation of curcumin monohydrate in acetone with drinking water (33 °F), borated water (62 ppm) or salinated water (5% NaCl) gives curcumin monohydrate.

If the recrystallisation solvent is ethyl acetate or ethyl acetate/acetone anhydrous curcumin is always obtained irrespective of whether the process starts with anhydrous curcumin or monohydrate.

Alternatively, recrystallisation of curcumin anhydrous can be performed in toluene, isopropanol, methanol, tert-butyl ether, ethanol or methyl ethyl ketone. X-ray diffraction does not indicate any differences between the solvents.

The results suggest that the purity of the curcumin, salt content and solvent are critical parameters for obtaining the hydrate or the anhydrous form and the main conclusions are:
- Crystallisation with acetone/water is highly variable,
- Crystallisation in ethyl acetate is adequate for obtaining the anhydrous form,
- The hydrated form is formed on crystallising with acetone and water with a high salt level.

Elemental analysis, X-ray diffraction, IR spectra, thermograms and solubility studies corroborate that curcumin monohydrate is a hydrate and not curcumin with absorbed water.

The main X-ray diffraction bands of curcumin monohydrate after crystallisation in acetone/drinking water are:

| Batch 009/2011 | | |
|---|---|---|
| (Å) | 2θ | % |
| 6.39 | 13.84 | 100.0 |
| 5.26 | 16.83 | 63.8 |
| 4.94 | 17.93 | 15.6 |
| 4.52 | 19.62 | 11.3 |
| 4.19 | 21.18 | 24.2 |
| 3.76 | 23.63 | 15.7 |
| 3.41 | 26.13 | 33.4 |
| 3.36 | 26.54 | 37.5 |
| 3.25 | 27.41 | 22.9 |
| 2.93 | 30.47 | 10.1 |

These are different from the bands of the anhydrous form recrystallised in various solvents: isopropanol, methanol, tert-butyl methyl ether, ethyl acetate, ethanol, methyl ethyl ketone and toluene.

| REF | | | IPA | MeOH | TBME | EthAc | EtOH | MEK | TOL |
|---|---|---|---|---|---|---|---|---|---|
| (Å) | 2θ | % | % | % | % | % | % | % | % |
| 11.11 | 7.9 | 11.0 | 14.5 | 12.5 | 10.5 | 11.4 | 12.4 | 17.9 | 12.2 |
| 9.92 | 8.91 | 18.2 | 43.6 | 45.1 | 25.7 | 23.6 | 36.8 | 37.6 | 40.7 |
| 7.21 | 12.25 | 4.1 | 4.5 | 11.4 | 7.0 | 6.6 | 9.0 | 6.3 | 11.5 |
| 6.10 | 14.51 | 10.1 | 15.8 | 20.4 | 15.0 | 13.5 | 17.9 | 19.6 | 20.4 |
| 5.56 | 15.93 | 11.9 | 14.8 | 15.3 | 11.5 | 11.8 | 13.4 | 20.9 | 12.2 |
| 5.14 | 17.25 | 100.0 | 100.0 | 100.0 | 100.0 | 100.0 | 100.0 | 100.0 | 100.0 |
| 4.98 | 17.84 | 7.1 | 12.8 | 14.6 | 10.4 | 8.8 | 13.2 | 12.1 | 14.3 |
| 4.87 | 18.19 | 8.4 | 10.6 | 22.9 | 16.1 | 11.1 | 16.4 | 14.5 | 22.7 |
| 4.70 | 18.73 | 6.3 | 5.4 | 12.9 | 10.0 | 8.9 | 9.8 | 8.5 | 11.5 |
| 4.56 | 19.45 | 6.7 | 6.3 | 14.3 | 10.2 | 8.4 | 10.4 | 8.8 | 13.1 |
| 4.19 | 21.16 | 21.5 | 16.7 | 25.5 | 24.7 | 18.6 | 21.0 | 25.4 | 23.6 |
| 3.89 | 22.86 | 5.0 | 8.3 | 10.0 | 4.9 | 6.6 | 8.6 | 8.6 | 9.7 |
| 3.81 | 23.33 | 16.2 | 18.7 | 38.1 | 27.1 | 21.0 | 28.3 | 23.7 | 34.9 |
| 3.75 | 23.73 | 13.4 | 15.3 | 28.8 | 22.5 | 16.9 | 22.6 | 21.1 | 24.8 |
| 3.60 | 24.73 | 9.4 | 9.5 | 31.3 | 21.8 | 14.5 | 22.7 | 16.7 | 28.4 |
| 3.46 | 25.71 | 9.4 | 10.4 | 26.1 | 17.9 | 11.3 | 17.6 | 15.7 | 25.8 |
| 3.33 | 26.71 | 13.9 | 12.0 | 22.2 | 21.0 | 14.2 | 18.5 | 16.7 | 22.3 |
| 3.24 | 27.48 | 7.3 | 7.3 | 20.1 | 14.1 | 10.4 | 14.3 | 10.8 | 16.9 |
| 3.16 | 28.21 | 16.9 | 12.5 | 17.4 | 16.4 | 12.9 | 15.2 | 17.9 | 14.1 |
| 3.05 | 29.30 | 7.5 | 9.3 | 15.0 | 11.0 | 8.3 | 12.1 | 13.6 | 14.9 |

Elemental analysis of curcumin monohydrate gives 65.79% carbon and 5.75% hydrogen compared to 68.37% carbon and 5.46% hydrogen for the anhydrous form.

The thermogram of curcumin monohydrate shows the loss of one molecule of water at around 50 °C compared to anhydrous curcumin. The IR spectra of the anhydrous and monohydrate forms are also different.

The solubility of curcumin monohydrate in ethyl acetate is higher than the solubility of anhydrous curcumin. On dissolving curcumin monohydrate and refluxing in ethyl acetate, followed by subsequent cooling, anhydrous curcumin is obtained.

Converting anhydrous curcumin to curcumin monohydrate is performed by crystallisation with acetone and water with a high salt content, for example, 5% sodium chloride or in water doped with boron (III).

Curcumin monohydrate shows similar photosensitising activity to anhydrous curcumin *in vitro.* Curcumin monohydrate at a concentration over 0.5 µg/ml with UVA or visible light (1 J/cm²) exhibits inhibition of the growth of HaCaT cells and human keratinocytes in culture and induces apoptosis without harming the cell membrane. Curcumin monohydrate can be mixed with pharmaceutically acceptable excipients for the preparation of pharmaceutical forms for oral, sublingual, intranasal, anal, otic, transdermal, topical, ocular, intraperitoneal, subcutaneous, intrathecal, vaginal, intravenous or intramuscular administration.
Figure 1. Sample of the IR spectrum of anhydrous curcumin.
Figure 2. Sample of the IR spectrum of curcumin monohydrate.
Figure 3. Sample of the thermogram of anhydrous curcumin.
Figure 4. Sample of the thermogram of curcumin monohydrate.

### Example 1. Obtaining crystalline anhydrous curcumin

A suspension of tributyl borate (22.0 L, 81.5 mol), boron oxide (2.24 kg, 32.1 mol), butylamine (1.34 L, 13.6 mol) and vanillin (10.68, 70.2 mol) was prepared in ethyl acetate (34.0 L). The reaction mixture was heated to 70 °C. A solution of acetylacetone (3.40 kg, 34.0 mol) in ethyl acetate (5.0 L) was added drop by drop over 4 hours at 70 °C. The mixture was cooled to room temperature and the reaction allowed to proceed for 12-18 hours. The precipitated solid, which was the boron-curcumin complex, was filtered by centrifugation and washed with ethyl acetate. The residue (13.9 kg) was suspended in drinking water (33 °F, 96.0 L) and glacial acetic acid (4.90 L). The mixture was stirred for 30 minutes, filtered by centrifugation and washed with drinking water (10.0 L). The residue was suspended in acetone (45.0 L) and heated to reflux for 15 minutes. Drinking water (45.0 L) was added, keeping the solution under reflux. The mixture was clarified by membrane filtration and cooled to room temperature. The precipitated solids were filtered by centrifugation and washed with acetone (8.0 L). The residue obtained (12.4 kg) was dried under vacuum at 50 °C to give anhydrous curcumin as a yellow crystalline solid (8.3 kg, 64%).

Then the curcumin was recrystallised in ethyl acetate showing the same IR and X-ray diffraction spectra.

### Example 2. Obtaining curcumin monohydrate.

A suspension of tributyl borate (21.78, 80.6 mol), boron oxide (2.21 kg, 31.78 mol), butylamine (1.34 L, 13.6 mol) and vanillin (10.57 kg, 69.49 mol) was prepared in ethyl acetate (35.0 L). The reaction mixture was heated to 70 °C. A solution of acetylacetone (3.37 kg, 33.7 mol) in ethyl acetate (5.0 L) was added drop by drop over 4 hours at 70 °C. The mixture was cooled to room temperature and the reaction allowed to proceed for 12-18 hours. The precipitated solid, which was the boron-curcumin complex, was filtered by centrifugation and washed with ethyl acetate. The residue (13.8 kg) was suspended in drinking water (33 °F, 95.0 L) and glacial acetic acid (4.90 L). The mixture was stirred for 30 minutes, filtered by centrifugation and washed with drinking water (10.0 L). The residue was suspended in acetone (45.0 L) and heated to reflux for 15 minutes. Drinking water (45.0 L) doped with 62 ppm of boron (III) was added, keeping the solution under reflux. The mixture was clarified by membrane filtration and cooled to room temperature. The precipitated solids were filtered by centrifugation and washed with acetone (8.0 L). The residue obtained (12.2 kg) was dried under vacuum at 40 °C to give curcumin monohydrate as orange flakes (8.5 kg, 62%).

### Example 3. Conversion of curcumin monohydrate to anhydrous curcumin.

Five (5) grams of curcumin monohydrate were dissolved in 45 ml ethyl acetate under reflux. During heating, the solution changed colour before all the curcumin had dissolved and on cooling, anhydrous curcumin was precipitated, which was separated by filtration.

## Claims

1. An industrial process for synthesizing curcumin ,wherein the batch size is greater than 1 kg comprising:
a. Preparation of a suspension of an alkyl borate, boron oxide, butylamine and vanillin in ethyl acetate,
b. Addition of a solution of acetylacetone in ethyl acetate to the above solution drop by drop and reaction at 70°C,
c. Filtration of the solids formed on cooling,
d. Hydrolysis of the solids with an glacial acetic acid solution in water,
e. Filtration of curcumin formed and crystallization in organic solvents.

2. The process according to claim 1 for obtaining curcumin anhydrous wherein the curcumin is recrystallized in acetone/water.

3. A process for obtaining curcumin monohydrate according to claim 1 wherein the organic solvent is acetone/ water doped with boron (III).

4. A process for obtaining curcumin monohydrate according to claims 1 wherein the organic solvent is acetone/ water with 5% sodium chloride.

5. A process for obtaining curcumin anhydrous by obtaining curcumin monohydrate according to the process of claim 3 and re-crystallising the obtained curcumin in ethyl acetate.

6. A process for manufacturing a pharmaceutical composition comprising:
- the synthesis of curcumin according to claims 1-5 and
- the addition of suitable excipients.

## Patentansprüche

1. Ein industrielles Verfahren zur Synthese von Curcumin, wobei die Charge größer als 1 kg ist, Folgendes umfassend:
a. Herstellung einer Suspension eines Alkylborats, Boroxids, Butylamins und Vanillins in Ethylacetat,
b. Zugabe einer Lösung von Acetylaceton in Ethylacetat zur obigen Lösung, tropfenweise und mit Reaktion bei 70° C,
c. Filtration der beim Abkühlen gebildeten Feststoffe,
d. Hydrolyse der Feststoffe mit einer Eisessiglösung in Wasser,
e. Filtration des gebildeten Curcumin und Kristallisation in organischen Lösungsmitteln.

2. Verfahren nach Anspruch 1 zur Gewinnung von wasserfreiem Curcumin, wobei das Curcumin in Aceton/Wasser umkristallisiert wird.

3. Ein Verfahren zur Gewinnung von Curcumin-Monohydrat nach Anspruch 1, wobei das organische Lösungsmittel mit Bor (III) dotiertes Aceton/Wasser ist.

4. Ein Verfahren zur Gewinnung von Curcumin-Monohydrat nach Anspruch 1, wobei das organische Lösungsmittel Aceton/Wasser mit 5 % Natriumchlorid ist.

5. Ein Verfahren zur Gewinnung von wasserfreiem Curcumin durch Gewinnung von Curcumin-Monohydrat gemäß dem Verfahren nach Anspruch 3 und Umkristallisieren des erhaltenen Curcumin in Ethylacetat.

6. Ein Verfahren zur Herstellung einer pharmazeutischen Zusammensetzung, Folgendes umfassend:
- die Synthese von Curcumin nach den Ansprüchen 1-5,
- die Zusage geeigneter Hilfsstoffe.

## Revendications

1. Un procédé industriel pour la synthétisation de la curcumine, dans lequel la taille de lot est supérieure à 1 kg, comprenant :
a. Préparation d'une suspension d'un borate d'akyle, oxyde de bore, butylamine et vanilline en acétate d'éthyle,
b. Addition d'une solution d'acétylacétone en acétate d'éthyle à la solution ci-dessus goutte à goutte et réaction à 70°C,
c. Filtration des solides formés dans le refroidissement,
d. Hydrolyse des solides avec une solution d'acide acétique glacial dans l'eau,
e. Filtration de la curcumine formée et cristallisation en solvants organiques.

2. Le procédé selon la revendication 1 pour l'obtention de curcumine anhydre dans lequel la curcumine est recristallisée en acétone/eau.

3. Un procédé pour l'obtention de curcumine monohydratée conformément à la revendication 1, où le solvant organique est de l'acétone/eau dopée au bore (III).

4. Un procédé pour l'obtention de curcumine monohydratée conformément à la revendication 1, où le solvant organique est de l'acétone/eau avec 5 % de chlorure de sodium.

5. Un procédé pour l'obtention de curcumine anhydre en obtenant de la curcumine monohydratée conformément au procédé de la revendication 3, et recristallisation de la curcumine obtenue en acétate d'éthyle.

6. Un procédé pour la fabrication d'une composition pharmaceutique comprenant :
- La synthèse de curcumine conformément aux revendications 1-5 et
- L'addition d'excipients appropriés.
